# DEMANDE DE BREVET EUROPEEN

(11) **EP 2 016 974 A1**
(43) Date de publication de la demande: **21.01.2009**
(21) Numéro de dépôt: 08290644.7
(22) Date de dépôt: 01.07.2008
(51) Int. Cl.: A61N 1/05

(54) **Sonde à électrodes multiples et système pour neurostimulation électrique profonde comportant une telle sonde**

(30) Priorité: 03.07.2007 FR 0704804
(71) Demandeur: COMMISSARIAT A L'ENERGIE ATOMIQUE, 75015 Paris (FR)
(72) Inventeur: Benabid, Alim Louis, 38240 Meylan (FR); Sauter-Starace, Fabien, 38170 Seyssinet-Pariset (FR); Caillat, Patrice, 38000 Grenoble (FR)
(74) Mandataire: Priori, Enrico

(57) **Abrégé**

Sonde (1) pour neurostimulation électrique profonde comportant :
- une tige (10) en matériau biocompatible ; et
- un plateau (20), également en matériau biocompatible, fixé à une extrémité de ladite tige (10) et aligné avec elle ;
- dans lequel ledit plateau (20) présente deux faces principales (21, 22) et une pluralité d'électrodes (31, 32) disposées sur au moins l'une desdites deux surfaces principales (21, 22) suivant une configuration bidimensionnelle, lesdites électrodes (31, 32) étant reliées à des éléments conducteurs (33) disposés à l'intérieur ou sur une surface de ladite tige (10).

Système de neurostimulation électrique profonde comportant :
- un générateur d'impulsions électriques pour neurostimulation électrique profonde (100) ; et
- au moins une sonde (1) du type décrit ci-dessus, dont les électrodes (31, 32) sont raccordées électriquement audit générateur d'impulsions électriques (100).

## Description

L'invention porte sur une sonde pour neurostimulation électrique profonde, et plus particulièrement pour électrostimulation cérébrale profonde. L'invention porte également sur un système pour neurostimulation électrique profonde comportant au moins une telle sonde.

La stimulation cérébrale profonde (« deep brain stimulation » en langue anglaise) est une technique thérapeutique comportant l'implantation d'un appareil médical connu comme stimulateur cérébral, qui envoie des impulsions électriques à des parties spécifiques du cerveau. Par exemple, la stimulation des noyaux de thalamus ou de l'hypothalamus peut être utilisée pour traiter des désordres moteurs tels que des tremblements, provoqués notamment par le syndrome de Parkinson ; voir à ce propos les articles suivants :
- P. Krack, P Pollak, P. Limousin, A. Benazzouz et A. L. Benabid, The Lancet, vol 350, 6 décembre 1997 ; et
- A.L. Benabid et al., Acta Neurochirurgica suppl. Vol. 58, pages 39 - 44, 1993.

Il est également envisagé de stimuler le noyau hypothalamique postérieur pour traiter les céphalées en grappe, la matière grise périaqueducale pour atténuer la douleur et l'hypothalamus ventromédian pour soigner certains cas d'obésité ; voir à ce propos les articles suivants :
- A. Takaki, S. Aou , Y. Oomura, E. Okada, T. Hori, « Feeding suppression elicted by electrical and chemical stimulation of monkey hypothalamus », Am. J. Physiol. 262 (Regulatory Integrative Comp. Physiol. 31) R5866-R594, 1992.
- K. Sano, Y. Mayanagi, H. Sekino, M. Ogashiwa et al « Results of stimulation and destruction of the posterior hypothalamus in man », J Neurosurg 33, décembre 1970, pages 689-707.
- C. Bielajew, J. Stenger, D. Schindler, « Factors that contribute to reduce weight gain following chronic ventromedial stimulation », Behav. Brain Res. 62 (1994), pages143-146. Et :
- F. Brown, R. Fessler, J. Rachlin, S. Mullan, « Changes in food intake with electrical stimulation of the ventromedial hypothalamus in dogs » J. Neurosurg. 60, pages 1253-1257(1984).

Récemment, une étude a montré que la stimulation électrique du cortex cingulaire subgénual, et plus précisément de l'aire de Brodmann 25 (CG25), peut être utilisée pour traiter des formes particulièrement graves et résistantes au traitement de dépression clinique [H.S. Mayberg et al. « Deep Brain Stimulation for Treatment-Resistant Depression », Neuron, Vol. 45, pages 651-660, 3 mars 2005]. La méthode de stimulation préconisée est celle du cortex subgénual CG25 par application directe, inter hémisphérique, d'électrodes de surface similaires à celles utilisées pour la stimulation des cortex prémoteurs dans des indications de douleurs rebelles.

Dans tous les cas, une intervention de stimulation cérébrale profonde comporte l'insertion, dans le crâne du patient, d'une sonde souple guidée par une canule et/ou un stylet rigide, jusqu'à que la pointe de ladite sonde atteigne la région du cerveau à stimuler. A proximité de sa pointe, la sonde comporte des électrodes (généralement quatre), qui sont reliés par un câble sous-cutané à un appareil de génération d'impulsions, implanté à son tour sous la peau du patient comme un stimulateur cardiaque conventionnel. La canule et/ou le stylet sont extraits du crâne du patient après avoir servi à l'introduction de la sonde, alors que cette dernière reste en place pendant une durée qui peut atteindre plusieurs années.

Une description plus détaillée de la procédure d'implantation d'une sonde pour stimulation cérébrale profonde est fournie par le document « Medtronic - DBS^{™} Lead Kit for Deep Brain Stimulation 3387 3389 - Implant manual » de la société Medtronic Inc., téléchargeable du site Internet : http://www.medtronic.com/physician/activa/downloadablefiles/197928_b_006. pdf.

Des sondes pour neurostimulation électrique profonde de type conventionnel sont décrites, par exemple, dans le document précité de la société Medtronic, ainsi que dans le document US 6,512,958.

La forme rectiligne de la sonde est imposée par la nécessité de rendre son insertion la moins traumatique possible pour le patient. Cependant, elle a le défaut de ne permettre que la stimulation d'une très petite région de tissu cérébral, alors que, afin d'obtenir une meilleure efficacité du traitement, il serait souhaitable de pouvoir agir sur une cible de plus grand volume. L'implantation de plusieurs sondes, visant des points distincts d'une même région cible de volume relativement important, est possible, mais comporte une multiplication des risques et des effets collatéraux de l'intervention.

Un but de l'invention est donc de procurer une sonde pour neurostimulation électrique profonde permettant, au moins dans le cadre de certaines applications, telles que la stimulation de la région CG25, d'obtenir une meilleure efficacité du traitement tout en minimisant ses risques et ses effets collatéraux.

Conformément à l'invention, ce but est atteint par une sonde pour neurostimulation électrique profonde caractérisée en ce qu'elle comporte : une tige en matériau biocompatible ; et un plateau, également en matériau biocompatible, fixé à une extrémité de ladite tige et aligné avec elle ; dans lequel ledit plateau présente deux faces principales et une pluralité d'électrodes disposées sur au moins l'une desdites deux surfaces principales suivant une configuration bidimensionnelle, lesdites électrodes étant reliées à des éléments conducteurs disposés à l'intérieur ou sur une surface de ladite tige.

Selon des modes de réalisation particuliers de l'invention :
- Ledit plateau peut présenter une pluralité d'électrodes disposées sur ses deux faces principales opposées.
- Le nombre d'électrodes disposées sur ladite ou chaque face principale dudit plateau peut être compris entre 1 et 40 et de préférence entre 5 et 20.
- Ladite tige peut être sensiblement rectiligne.
- Ladite tige et ledit plateau peuvent être réalisés, au moins en partie, en un matériau biocompatible choisi parmi les silicones, les siloxanes, le polyuréthane, le polyvinyle chlorure, le benzocyclobutène (BCB), les polyimides et le parylène.

Ladite tige et ledit plateau peuvent être réalisés d'une seule pièce.
- Ladite sonde peut présenter une longueur totale comprise entre 4 et 10 cm et de préférence entre 5 et 8 cm.
- Ladite sonde peut présenter une forme sensiblement planaire, avec une épaisseur comprise entre 25 µm et 3 mm.. et de préférence entre 50µm et 2 mm.
- Lesdites faces principales du plateau peuvent présenter une surface comprise entre 10 mm² et 500 mm² et de préférence entre 20 mm² et 450 mm².
- Ledit plateau peut présenter une forme : en faucille, elliptique, ou épousant le contour d'une zone CG25 d'un cerveau humain.
- Au moins ladite tige peut présenter une section creuse avec une lumière s'étendant longitudinalement, auquel cas ladite sonde peut comporter un stylet rigide introduit de manière amovible à l'intérieur de ladite lumière.
- Ladite tige peut présente, au repos, une forme non rectiligne et être susceptible de se redresser de manière réversible lors de l'introduction dudit stylet, pour retrouver sa forme non rectiligne lors de l'extraction de ce dernier.
- Ledit plateau peut être creux et susceptible de s'étirer de manière réversible lors de l'introduction dudit stylet pour retrouver sa forme d'origine lors de l'extraction de ce dernier.
- Ladite sonde peut comporter des poutres de rigidification longitudinales intégrées à ladite tige. Lesdites poutres de rigidification longitudinales peuvent être réalisées en un matériau choisi parmi les métaux et alliages non magnétiques et les fibres de carbone.
- Lesdites électrodes peuvent présenter un revêtement en nanotubes de carbone électriquement conducteurs, et plus particulièrement de nanotubes multiparois, de préférence avec ramifications, recouvrant des pastilles en silicium reportées sur la surface desdites électrodes.

Un autre objet de l'invention est un système de neurostimulation électrique profonde comportant : un générateur d'impulsions électriques pour neurostimulation électrique profonde ; et au moins une sonde telle que décrite ci-dessus, dont les électrodes sont raccordées électriquement audit générateur d'impulsions électriques.

D'autres caractéristiques, détails et avantages de l'invention ressortiront à la lecture de la description faite en référence aux dessins annexés donnés à titre d'exemple et qui représentent, respectivement :
- la figure 1, une vue en plan d'une sonde selon l'invention ;
- la figure 2, une vue en section longitudinale, selon la ligne II-II, d'une sonde selon un premier mode de réalisation de l'invention ;
- la figure 3, une vue en section transversale, selon la ligne III-III, d'une sonde selon ledit premier mode de réalisation de l'invention ;
- la figure 4, une vue en section transversale d'une sonde selon un deuxième mode de réalisation de l'invention ; et
- la figure 5, un schéma de principe d'un système de neurostimulation électrique profonde comportant une sonde selon l'invention, implantée dans le cerveau du patient afin de stimuler la région CG25 de son cortex cingulaire subgénual.

Une sonde 1 selon l'invention comporte essentiellement deux parties : une tige sensiblement rectiligne 10 et un plateau 20 en forme de faucille ou haricot, fixé à une extrémité dite distale de ladite tige 10 et aligné avec elle, de manière à en constituer une prolongation. Aussi bien la tige 10 que le plateau 20 sont réalisés en un matériau biocompatible, de préférence relativement souple, tel qu'une silicone, un siloxane, le polyuréthane, le polyvinyle chlorure, le benzocyclobutène (BCB), polyimide et le parylène. La tige 10 et le plateau 20 sont de préférence réalisés d'une seule pièce, mais cela n'est pas essentiel.

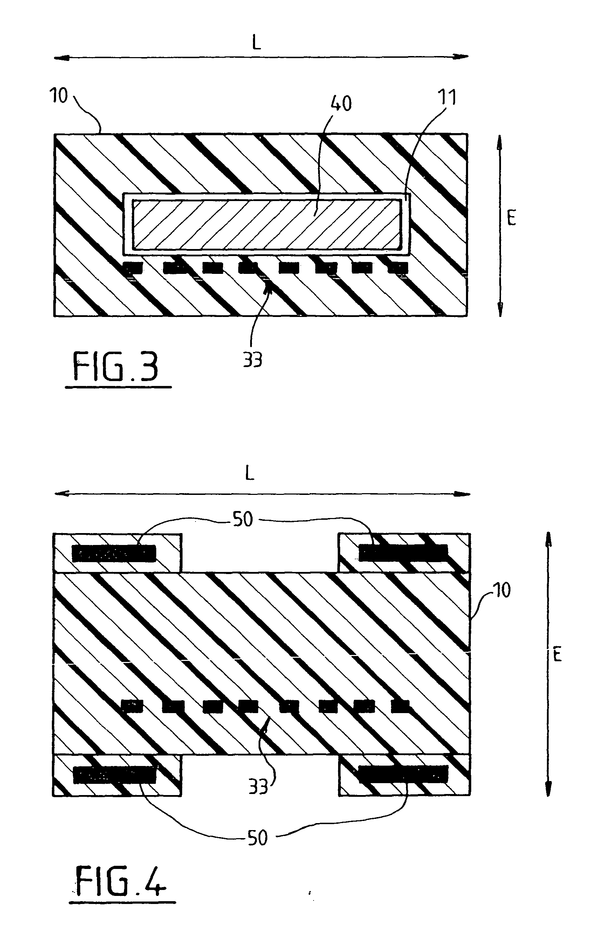

La sonde 1 présente une structure sensiblement planaire. La tige 10 présente une section au moins approximativement rectangulaire, comme cela est représenté sur les figures 3 et 4, avec une épaisseur E généralement comprise entre 25 µm et 3 mm, et de préférence entre 50 µm et 2 mm. et une largeur L généralement comprise entre 10 mm et 40 mm, et de préférence entre 15 et 30 mm. En variante, la tige 10 peut également présenter une forme sensiblement cylindrique, avec un rayon généralement compris entre 0,5 et 2 mm. La tige 10 peut être située au milieu du plateau 20, comme dans l'exemple des figures, ou bien sur un bord de ce dernier, en fonction de la géométrie de la zone à stimuler et des contraintes de rigidité à l'insertion.

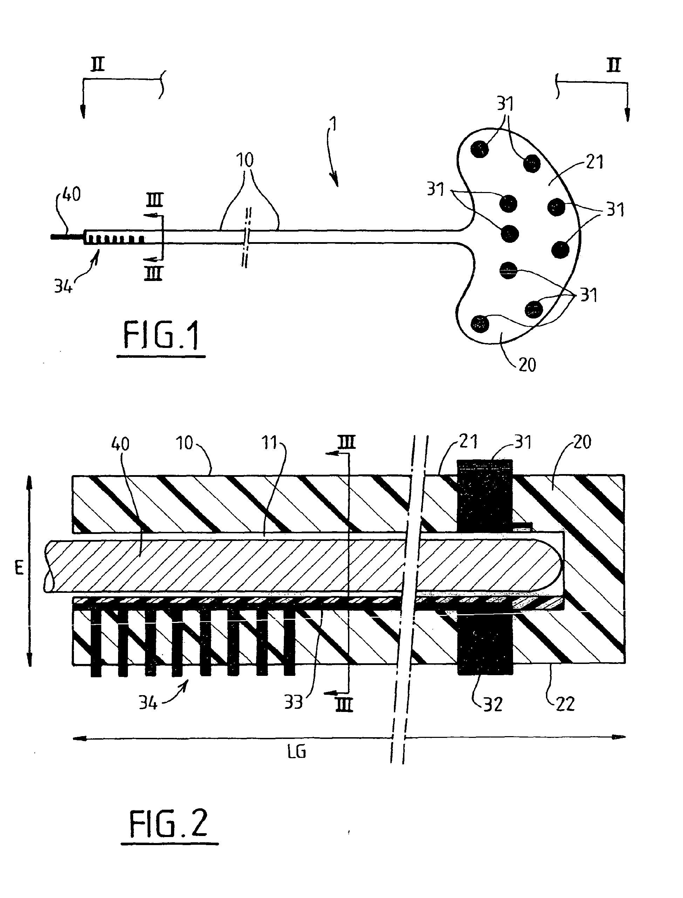

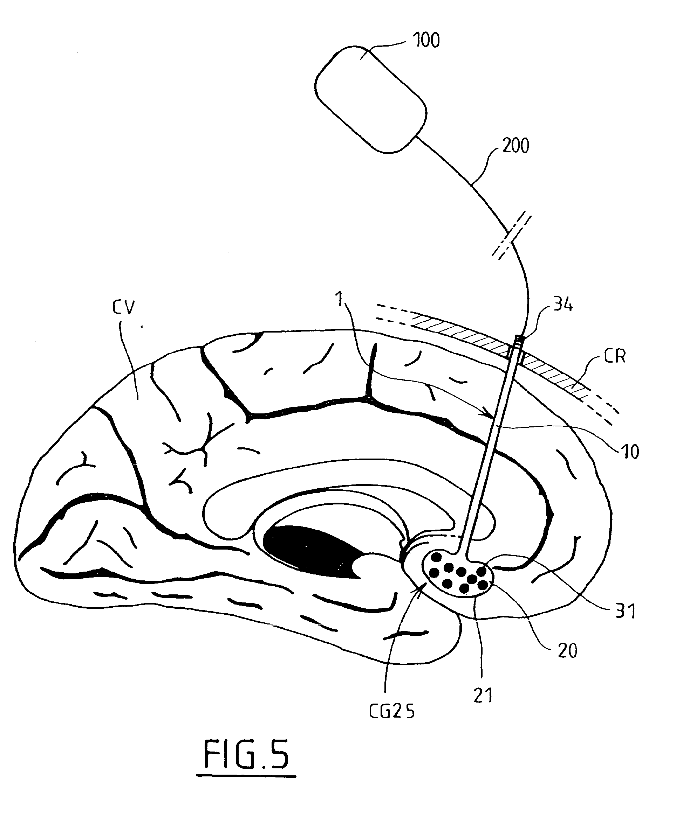

Le plateau 20 présente également une structure sensiblement planaire, avec une épaisseur du même ordre de grandeur de celle de la tige 10, et de préférence avec la même épaisseur. Le plateau présente deux faces principales opposées, 21 et 22, qui dans l'exemple des figures 1 et 5 ont une forme en faucille, dont la surface est approximativement comprise entre 10 mm² et 750 mm² et de préférence entre 100 mm² et 450 mm². La forme en faucille ou haricot a été choisie car elle correspond approximativement à celle de l'aire CG25 à stimuler. En variante on peut choisir une forme plus complexe, épousant de manière précise le contour de l'aire CG25, ou inversement une forme plus simple, elliptique ou circulaire. Des formes différentes peuvent être prévues, notamment pour la stimulation d'autres zones du cerveau. En général, il est avantageux que le plateau 20 s'étende sur toute la surface de la zone à stimuler, voire au-deià.

La longueur LG de la sonde 1 est de préférence comprise entre 3 et 8 cm. Cette longueur doit être suffisante pour permettre d'atteindre le tissu cible à stimuler, par exemple la région CG25 du cerveau humain, sans dépasser de manière excessive de la surface du corps du patient.

Des électrodes 31, 32 font saillie des deux faces principales opposées 21, 22 du plateau 20. Ces électrodes, réalisés en un matériau conducteur biocompatible tel que du platine, sont disposées suivant une configuration bidimensionnelle. La figure 1 montre par exemple une sonde 1 dont le plateau 20 présente neuf électrodes sur chacune de ses faces principales, disposées selon un motif permettant une stimulation approximativement uniforme de l'aire CG25. Le nombre d'électrodes sur chaque face principale 21, 22 peut être compris, par exemple, entre 1 et 40, et de préférence entre 5 et 20.

Les dimensions des électrodes 31, 32 sont déterminées en fonction du courant de stimulation prévu, sachant qu'il n'est pas souhaitable de dépasser une densité de charge de 30 µC/cm² par impulsion.

En variante, une seule des faces principales du plateau 20 peut être munie d'électrodes, de manière à ne produire qu'une électrostimulation unilatérale du tissu cible. Une électrostimulation bilatérale peut également être obtenue en utilisant deux sondes de ce type disposées dos à dos.

Les électrodes 31, 32 sont connectées à des éléments conducteurs 33 qui s'étendent longitudinalement le long de la tige 10, jusqu'à des contacts électriques externes 34, situés au niveau de l'extrémité dite proximale, opposée au plateau 20, de ladite tige. Comme représenté sur la figure 5, lorsque la sonde 1 est implantée dans le cerveau CV d'un patient, les contacts électriques externes dépassent de sa boîte crânienne CR, et permettent de relier électriquement les électrodes 31, 32 à un générateur d'impulsions électriques pour neurostimulation électrique profonde 100 par l'intermédiaire d'un câble sous-cutané 200.

Les éléments conducteurs 33 peuvent être disposés à i'intérieur de ia tige 10 ou sur sa surface ; dans ce dernier cas, cependant, ils doivent être recouverts d'une couche d'isolant électrique et de passivation.

La sonde 1 selon l'invention, et en particulier sa tige 10, doit être suffisamment souple pour ne pas provoquer des lésions internes lorsqu'elle est implantée à demeure, par exemple dans le cerveau d'un patient. En même temps, son insertion nécessite d'une rigidité suffisante.

D'une manière connue en soi, ces exigences contradictoires sont résolues en utilisant une sonde 1, ou au moins une tige 10, ayant une section creuse, avec une lumière longitudinale 11 dans laquelle peut être introduit de manière amovible un stylet de rigidification 40, destiné à être extrait de la sonde 1 après son introduction dans le corps d'un patient.

Selon une variante de l'invention, la tige 10 peut présenter une préforme non rectiligne. Une telle tige se redresse de manière réversible lors de l'insertion du stylet 40, ce qui facilite son implantation ; puis, au retrait du stylet elle reprend sa forme originaire non rectiligne. Cela permet d'atteindre des zones du cerveau qui seraient autrement difficiles d'accès, et cela en minimisant les lésions du parenchyme provoquées par l'insertion de la sonde.

Il est également possible de réaliser une sonde présentant un plateau 20 creux et de préférence pourvu d'entailles longitudinales. Lorsque le stylet 40 prend appui sur l'extrémité distale de la sonde, un tel plateau s'étire, en devenant plus long et plus étroit. La sonde prend alors une forme quasiment rectiligne, ce qui permet de minimiser la taille de l'incision qui doit être pratiquée dans la boîte crânienne pour son insertion, ainsi que les lésions du parenchyme provoquées par ladite insertion. Lors du retrait du stylet, le plateau 20 reprend sa forme d'origine pour stimuler efficacement la région cible du cerveau.

Des sondes de stimulation cérébrale dont la forme est temporairement modifiée par l'insertion d'un stylet de rigidification sont décrites plus en détail dans la demande de brevet FR 07/01353 déposée le 26 février 2007 par le présent Demandeur.

Dans certains cas, il peut être préférable d'utiliser une sonde 1 pourvue en elle-même d'une certaine rigidité, mais ayant une enveloppe relativement souple. Cela peut être obtenu en rigidifiant une sonde principalement réalisée en matière souple par des poutres longitudinales intégrées 50, réalisées par exemple en tantale ou en titane, ou tout autre métal biocompatible et compatible avec l'IRM (imagerie par résonance magnétique) ou en fibres de carbone. De préférence, les poutres 50 sont éloignées de l'axe de flexion de la tige 10, ce qui revient à accroître la rigidité des poutres pour un même volume.

Dans le mode de réalisation de la figure 4, les poutres 50 rigidifient la sonde 1 de manière suffisante à permettre son introduction sans besoin d'un stylet 40 ; la sonde a par conséquent une section pleine, qui ne comporte pas de lumière. Il est également possible d'envisager un mode de réalisation intermédiaire, dans lequel les poutres 50 n'assurent pas une rigidité suffisante, et un stylet amovible 40 est utilisé pour l'insertion de la sonde, comme dans lé mode de réalisation de la figure 3.

D'un point de vue technologique, des sondes selon l'invention peuvent être obtenues à partir de différents procédés de fabrication.

Pour des sondes d'une épaisseur supérieure à 100 µm et jusqu'à quelques millimètres, afin de fabriquer les électrodes 31, 32, les éléments conducteurs 33 et les contacts électriques externes 34 on utilisera de préférence des étapes de photolithographie et gravure de métal sur élastomère de type siloxane ou par dépôt d'encres conductrices ou par dépôt de nanopoudres suivi d'une opération de frittage laser qui permet d'atteindre une bonne conduction électrique, ces étapes étant suivies par une passivation avec silicone photosensible ou parylène ouvert localement par gravure plasma ou laser.

Pour des sondes d'épaisseur comprise entre 10 et 100 µm environ, on partira d'un polymère photosensible ou non (géométrie obtenue par lithographie ou par gravure plasma), tel qu'un polyimide ou du benzocyclobutène (BCB), déposé par centrifugation, sur lequel des motifs conducteurs en deux dimensions constituant les électrodes 31, 32, les éléments conducteurs 33 et les contacts électriques externes 34 sont réalisés par photolithographie ou également par dépôt d'encres conductrices ou par dépôt de nanopoudres. Le premier procédé (lithographie) permet d'atteindre des résolutions de l'ordre du micron alors que les suivants sont limités à des motifs de plus de 10 µm. Une passivation est obtenue en déposant une deuxième couche du même polymère. Une section creuse peut être obtenue à l'aide de couches sacrificielles, comme décrit dans l'article de Kee Keun Lee, Jiping He, Ryan, Clement, Stephen Massia et Bruce Kim, publié dans la revue « Biosensors and bioelectronics », numéro 20, pages 404 à 407, 2004, ou bien en réalisant des scellements après photo-polymérisation. C'est dans le cas de ces sondes très fines que l'utilisation de poutres 50 de renfort s'avère plus intéressant.

Dans les deux cas, les éléments conducteurs 33 peuvent être réalisés en métal, oxyde d'indium et aluminium (ITO) ou graphite, et être texturés par des méthodes issues des technologies des semi-conducteurs ou des circuits imprimés.

Afin d'accroître la biocompatibilité des électrodes de stimulation et/ou d'enregistrement, on pourra utiliser un report de pastilles de silicium aminci revêtues de nanotube de carbones. Les propriétés électriques des nanotubes de carbone (multiparois, avec ou sans ramification) montrent un accroissement très significatif du rapport signal sur bruit par une diminution de l'impédance de l'électrode (propriété liée à l'augmentation de la surface développée). Ces performances sont recherchées pour une électrode d'enregistrement de l'activité corticale dans la zone de stimulation à l'instar d'un électrcorticogramme (analogue à un électroencéphalogramme réalisé sur le cortex).

En stimulation les électrodes comportant des nanotubes de carbones présentent aussi des avantages. Les caractérisations électrochimiques de ces électrodes montrent que la quantité de charges injectables est bien supérieure à celle d'une électrode en Nitrure de Titane ou en platine (exemple de caractérisation faite par le CEA/Léti : la capacité surfacique des électrodes avec nanotubes de carbone a été mesurée à 1,3-10⁻² F/cm² contre 5,0·10⁻⁴ F/cm² pour ies mêmes électrodes sans nanotubes). L'utilisation des nanotubes de carbone apporte donc un avantage significatif aux électrodes de stimulation. De plus, le document US7162308 présente les avantages de la croissance des nanotubes de carbone en matière de biocompatibilité.

L'utilisation d'une sonde selon l'invention pour traiter certaines formes de dépression clinique est illustrée sur la figure 5. Dans ces formes de dépression, la région 25 du cortex cingulaire subgénual, CG25, est hyperactive, et cette hyperactivité peut être modérée par une stimulation électrique chronique. Chaque hémisphère du cerveau CV est pourvu d'une région CG25 propre, qui est limitrophe de la scissure interhémisphérique (plan de la coupe coronale de la figure). En général, il est souhaitable de stimuler simultanément les régions des deux hémisphères, mais une stimulation unilatérale pourra être préférée dans certains cas particuliers.

Une sonde 1 selon l'invention peut être introduite dans le crâne d'un patient par une ouverture de forme adaptée pratiquée dans sa boîte crânienne CR, son plateau 20 gisant dans le plan de la scissure interhémisphérique.

Plus précisément, le patient est positionné sur un cadre stéréotaxique, un robot stéréotaxique étant également positionné sur ledit cadre pour déterminer sur le scalp le site de l'incision cutanée, et le centre de la tréphine parasagittale droite qui sera réalisée pour l'abord de la scissure inter hémisphérique, sous microscope opératoire. Ensuite, une incision cutanée est réalisée parallèlement à la suture coronale, ce qui la rend plus esthétique qu'une incision parasagittale qui risquerait de déborder excessivement la ligne d'implantation des cheveux. Le scalp étant rétracté, une craniotomie est effectuée à l'aide d'une tréphine de 3cm, voire de 5cm de diamètre, du coté non dominant du patient, donc généralement à droite si ce dernier est droitier, tangentiellement à la ligne médiane. La dure-mère est incisée selon un diamètre parallèle à la ligne médiane et deux refends radiaux sont pratiqués en direction de la ligne médiane afin de donner le jour sur la scissure inter hémisphérique. Celle-ci est délicatement décollée de la faux du cerveau, par section des adhérences arachnoïdiennes, jusqu'au niveau de ia base de l'étage antérieur, en allant vers la partie la plus postérieure, subgénuale, du cortex frontal médio-basal L'installation stéréotaxique permet d'effectuer des contrôles radiologiques per-opératoires assurant la bonne localisation en regard de la cible déterminée.

La sonde 1 est ensuite avancée à l'intérieur de ladite scissure interhémisphérique, le cas échéant à l'aide d'un stylet de rigidification 40, jusqu'à que le plateau 20 muni des électrodes 31, 32 atteigne la région CG25 à stimuler. Après rétraction du stylet 40, un contrôle radiologique est alors réalisé et la dure mère suturée, la rondelle osseuse réappliquée et fixée à l'épicrâne par des points non résorbables, et laissant sortir les câbles des électrodes par le trait de scie de la tréphine. Les extrémités distales sont laissées sous le périoste de la même façon que pour les électrodes intra parenchymateuses.

Au troisième jour post-implantation des électrodes, une nouvelle IRM est pratiquée qui contrôle la position des électrodes et l'absence de complication hémorragique.

Sous anesthésie générale, au sixième jour post-implantation, un générateur d'impulsions électriques pour neurostimulation électrique profonde 100 est implanté sous la peau du patient dans la région sous claviculaire droite, et connecté aux contacts électriques externes 34 de la sonde par un double prolongateur 200 conduit de la tête à la clavicule par tunnélisations cutanées successives.

En variante, deux sondes 1 présentant des électrodes sur une seule face principale du plateau 20 peuvent être disposées dos à dos, et reliées à un même générateur d'impulsions 100, ou à deux générateurs individuels.

Bien qu'elle ait été décrite en relation avec son utilisation pour le traitement de la dépression clinique par stimulation de la région 25 du cortex cingulaire subgénual (CG25), l'invention peut s'appliquer plus généralement à la neurostimulation électrique profonde d'autres régions du cerveau, voire d'autres tissus, tels que la moelle épinière.

## Revendications

1. Sonde (1) pour neurostimulation électrique profonde comportant :
- une tige (10) en matériau biocompatible, présentant une rigidité suffisante pour permettre son insertion dans le cerveau d'un patient ; et
- un plateau (20), également en matériau biocompatible, fixé à une extrémité de ladite tige (10) et aligné avec elle ;
- **caractérisé en ce que** ledit plateau (20) présente deux faces principales (21, 22) et une pluralité d'électrodes (31, 32) disposées sur au moins l'une desdites deux surfaces principales (21, 22) suivant une configuration bidimensionnelle, lesdites électrodes (31, 32) étant reliées à des éléments conducteurs (33) disposés à l'intérieur ou sur une surface de ladite tige (10).

2. Sonde (1) selon la revendication 1, dans laquelle ledit plateau (20) présente une pluralité d'électrodes (31, 32) disposées sur ses deux faces principales opposées (21, 22).

3. Sonde (1) selon l'une des revendications précédentes, dans laquelle le nombre d'électrodes (31, 32) disposées sur ladite ou chaque face principale (21, 22) dudit plateau (20) est compris entre 1 et 40 et de préférence entre 5 et 20.

4. Sonde (1) selon l'une des revendications précédentes, dans laquelle ladite tige (10) est sensiblement rectiligne.

5. Sonde (1) selon l'une des revendications précédentes, dans laquelle ladite tige (10) et ledit plateau (20) sont réalisés, au moins en partie, en un matériau biocompatible choisi parmi les silicones, les siloxanes, le polyuréthane, le polyvinyle chlorure, le benzocyclobutène (BCB), les polyimides et le parylène.

6. Sonde (1) selon l'une des revendications précédentes, dans laquelle ladite tige (10) et ledit plateau (20) sont réalisés d'une seule pièce.

7. Sonde (1) selon l'une des revendications précédentes, présentant une longueur totale comprise entre 4 cm et 10 cm et de préférence entre 5 cm et 8 cm.

8. Sonde (1) selon l'une des revendications précédentes, présentant une forme sensiblement planaire, avec une épaisseur (E) comprise 25 µm et 3 mm, et de préférence entre 50 µm et 2 mm.

9. Sonde (1) selon l'une des revendications précédentes, dans laquelle lesdites faces principales (21, 22) du plateau (20) présentent une surface comprise entre 10 mm² et 500 mm² et de préférence entre 20 mm² et 450 mm².

10. Sonde (1) selon l'une des revendications précédentes, dans laquelle ledit plateau (20) présente une forme : en faucille, elliptique, ou épousant le contour d'une zone CG25 d'un cerveau humain.

11. Sonde (1) selon l'une des revendications précédentes, dans lequel au moins ladite tige (10) présente une section creuse avec une lumière (12) s'étendant longitudinalement.

12. Sonde (1) selon la revendication 11 comportant un stylet (40) rigide introduit de manière amovible à l'intérieur de ladite lumière (12) afin de conférer à la tige ladite rigidité suffisante pour permettre l'insertion de la sonde dans le cerveau d'un patient.

13. Sonde (1) selon la revendication 12, dans lequel ladite tige (10) présente, au repos, une forme non rectiligne et est susceptible de se redresser de manière réversible lors de l'introduction dudit stylet (40) pour retrouver sa forme non rectiligne lors de l'extraction de ce dernier.

14. Sonde (1) selon la revendication 12 ou 13, dans laquelle ledit plateau (20) est creux et susceptible de s'étirer de manière réversible lors de l'introduction dudit stylet (40) pour retrouver sa forme d'origine lors de l'extraction de ce dernier.

15. Sonde (1) selon l'une des revendications précédentes, comportant des poutres de rigidification longitudinales (50) intégrées à ladite tige.

16. Sonde (1) selon la revendication 15 dans laquelle lesdites poutres de rigidification longitudinales (50) sont réalisées en un matériau choisi parmi les métaux et alliages non magnétiques et les fibres de carbone.

17. Sonde (1) selon l'une des revendications précédentes dans laquelle lesdites électrodes (31, 32) présentent un revêtement en nanotubes de carbone électriquement conducteurs.

18. Sonde (1) selon la revendication 17 dans laquelle lesdits nanotubes de carbone sont des nanotubes multiparois recouvrant des pastilles en silicium reportées sur la surface desdites électrodes (31, 32).

19. Système de neurostimulation électrique profonde comportant :
- un générateur d'impulsions électriques pour neurostimulation électrique profonde (100) ; et
- au moins une sonde (1) selon l'une des revendications précédentes, dont les électrodes (31, 32) sont raccordées électriquement audit générateur d'impulsions électriques (100).

20. Système de neurostimulation électrique profonde selon la revendication 19, dans lequel ledit générateur d'impulsions électriques est adapté pour générer des impulsions aptes à traiter la dépression clinique par stimulation d'une zone CG25 du cerveau d'un patient humain.
